# EUROPEAN PATENT SPECIFICATION

(11) **EP 0 922 691 B1**
(45) Date of publication and mention of the grant of the patent: **28.04.2004**
(21) Application number: 98204110.5
(22) Date of filing: 03.12.1998
(51) Int. Cl.: C07C 45/50, C07C 29/16, B01J 31/40

(54) **Hydroformylation process**
Hydroformylierungsverfahren
Procédé d' hydroformylation

(30) Priority: 03.12.1997 EP 97309765
(43) Date of publication of application: 16.06.1999
(73) Proprietor: SHELL INTERNATIONALE RESEARCH MAATSCHAPPIJ B.V., 2596 HR Den Haag (NL)
(72) Inventor: Arnoldy, Peter, 1031 CM Amsterdam (NL); Bolinger, Cornelis Mark, Sugar Land, Texas 77478 (US); Moene, Robert, 1031 CM Amsterdam (NL); Drent, Eit, 1031 CM Amsterdam (NL); Van Gogh, Johan, 1031 CM Amsterdam (NL); Van Der Hulst, Cornelis Hyacinthus Maria, 1031 CM Amsterdam (NL)

(56) References cited:
- EP-A- 0 350 922
- WO-A-95/05354
- WO-A-97/15543
- FR-A- 2 232 363
- US-A- 4 300 002
- PERRY R.H.: 'PERRY'S CHEMICAL ENGINEERS' HANDBOOK SIXTH EDITION', 1984, MCGRAW-HILL

## Description

### Background of the invention

The invention relates to a hydroformylation process wherein ethylenically unsaturated compounds are contacted with carbon monoxide and hydrogen gas in a reaction zone in the presence of a solvent and a homogeneous catalyst based on a Group 8, 9 or 10 metal (referring to the IUPAC classification of elements in use in 1997).

The hydroformylation of ethylenically unsaturated compounds, to form products such as aldehydes and/or alcohols, is of considerable industrial importance. As is apparent from the literature, e.g., "New Syntheses with Carbon Monoxide" by J. Falbe (Springer-Verlag 1980; ISBN 0-387-09674-4) and "Carbonylation" by H.M. Colqhoun, D.J. Thompson and M.V. Twigg (Plenum Press 1991; ISBN 0-306-43747-3) a multitude of catalysts based on Group 8, 9 or 10 metals (Fe, Ru, Os; Co, Rh, Ir; Ni, Pd, and Pt) have been used in hydroformylation processes. The most important industrial hydroformylation processes are presently based on the Group 9 metals; Co and Rh. Extensive patent art is also present on hydroformylation processes based on the Group 10 metals, Ni, Pd and Pt.

For various reasons, not in the least the costs of replacing lost catalyst, the catalyst must be recovered from the hydroformylation product. Mere distillation of the hydroformylation product, however, may inactivate and hence destroy the catalyst. Catalysts that are prone to destruction are therefore separated, for instance, by extraction.

In International application WO 95/05354 a process is disclosed wherein a major portion of the metal component of the catalyst system is recovered by causing at the end of the reaction the crude product to form two immiscible layers, and separating the layer comprising the hydroformylation product from the layer comprising the catalyst. The product layer, however, will still contain active catalyst. This reference provides no teaching how that should be recovered.

From EP-A-0,350,922 a process is known for the separation and recovery of an aldehyde product from a non-aqueous hydroformylation reaction product composition. The process involves phase separation using added water or both added water and an added non-polar hydrocarbon compound. Comparative example 1 of this reference illustrates the inadequacy of recovery by mere phase separation, whilst improved phase separation is shown when water is added. However, it should be observed that this process is conducted in the presence of a water soluble hydroformylation catalyst system. Thus, ionically charged phosphorus ligands are used, which will -together with the metal complex- easily separate into the aqueous phase during the phase separation step. For catalyst systems based on non-ionically charged phosphorus ligands, which are the more common type ligands, this document provides no teaching either.

US-A-4300002 relates to a process for producing a polycyclic diol by a hydroformylation of a polycyclic bridged ring olefinic compound having another double bond, formyl or methylol group (at the other side of the bridgehead carbon) in the presence of a cobalt compound and a phosphine as a catalyst in the presence of a saturated hydrocarbon or aromatic hydrocarbon solvent. Said reaction mixture is cooled to result in a phase separation into solvent phase containing the catalyst and a polycyclic diol phase and the solvent phase is recycled into the reaction system.

US-A-3904547 relates to an improved process for recycling a cobalt hydroformylation catalyst from a hydroformylation reaction which comprises feeding a hydroformylation product to a separation zone wherein two phases are formed, a solvent phase containing the cobalt hydroformylation catalyst and another phase containing the catalyst and a hydroformylation compound, separating the catalyst from the hydroformylation compound in a countercurrent extractor, concentrating the catalyst in an evaporation zone and recycling it into the hydroformylation reaction.

Separation of the two phases is carried out in conventional vessels, such as tanks, towers or pressure. Examples 1 and 2 therein employ a settling chamber for phase separation.

WO-A-97/15543 relates to a process for the hydroformylation of ethylenically unsaturated compounds by reaction thereof with carbon monoxide and hydrogen in the presence of a catalyst and a solvent.

WO-A-97/15543 provides a class of compounds that allow phase separation without excessive cooling or use of large amounts thereof.

The inventors have set out to develop a hydroformylation process, based on non-ionically charged ligands, with essentially full catalyst recovery.

### Summary of the invention

Accordingly, a hydroformylation process is provided including the steps of:
(a) contacting one or more ethylenically unsaturated compound(s) in a reaction zone with carbon monoxide and hydrogen gas in the presence of a polar aprotic solvent and a homogeneous catalyst based on palladium and a non-ionically charged phosphorus ligand, to form a crude hydroformylation product;
(b) allowing a major portion of the solvent, wherein a major portion of the catalyst is dissolved, to separate and be withdrawn from the crude hydroformylation product in a phase separation zone; and
(c) removing from the separated hydroformylation product emerging from step (b) substantially all remaining catalyst dissolved therein with a non-aqueous extractant in an extraction zone;
wherein the separated solvent containing catalyst emerging from step (b) is fed to the reaction zone of step (a), and wherein the non-aqueous extractant used in step (c) is the same substance as the solvent used in step (a).

In step (c) as extractant the same substance is used as the solvent used in step (a), thereby avoiding contamination of recycle streams.

In a (fully integrated) process that uses recycle streams, the extraction step (c) is preferably carried out (in a multi-stage mode) with an amount of extractant that matches the amount of solvent that is dissolved in the hydroformylation product emerging from step (c). Note that after the separation step (b) the hydroformylation product is still saturated with solvent. For instance, at a temperature of 40 °C, 100 g of a sulfolane-saturated crude C11-C12 olefin-derived hydroformylation product will contain about 8 g of sulfolane and therefore about 8 g of sulfolane is to be used for extraction. Obviously, the amount of extractant need not be an exact match; the process may be adapted to cope with for instance 0.9 to 1.1 times that amount.

Extraction step (c) affords a catalyst-loaded extractant. The extractant from step (c) and solvent are of the same substance, therefore the catalyst-loaded extractant may be led to the reaction zone of step (a) and/or to the separation zone of step (b). The inventors found the latter embodiment to be beneficial as thereby substantially higher separation efficiencies could be realised in step (b).

The solvent-extracted hydroformylation product emerging from step (c) will contain solvent and/or extractant. Typically, the solvent and/or the extractant also needs removal. Distillation is one option, as the extracted hydroformylation product is now freed from valuable catalyst. However, this manner of separation may be less applicable when the solvent, the extractant and the product have similar boiling points. Therefore, removal by washing is preferred.

### Detailed description of the invention

Obviously, in case the solvent and/or the extractant are removed by washing, the problem should not be augmented by the presence of the medium used for washing. In other words, the solubility of the hydroformylation product into the medium should be less than into the solvent and/or extractant. Furthermore, the washing medium should preferably be a harmless impurity without undesired side-effects when becoming part of the recycle streams. Moreover, the medium should be cheap.

Water was found to be quite effective as washing medium. Thus, a step (d) is preferably added to the process of the invention that comprises:
(d) removing from the extracted hydroformylation product emerging from step (c) substantially all remaining solvent and/or extractant dissolved therein with a washing medium, that is preferably water, in a washing zone, separating the solvent and/or extractant from the washing medium emerging from the washing zone and optionally reusing the solvent and/or extractant.

Preferably the water-washing is conducted (in a multi-stage mode) at temperatures in excess of 60 °C, more preferably in excess of 70 °C and at a phase ratio of crude versus medium varying from 1:0.2 to 1:1 w/w. The lower temperature is important to avoid emulsification.

In the present invention, wherein the extractant and the solvent are of the same substance, the isolated extractant is preferably reused as extractant in step (c). Surprisingly, this small amount of extractant is sufficient to effectively remove substantially all the remaining catalyst from the separated hydroformylation product emerging from step (b).

The extraction may be conducted in a column equipped with a rotary-disk contactor (cf. Perry's Chemical Engineers' Handbook, 6th ed., p. 21-77 and further) or a packed bed column. The packed bed column is more efficient in the present process, avoiding undesired emulsification, and is hence preferred. Highest extraction efficiencies may be realised using a packed bed column with structured packing.

The separated solvent emerging from step (b) contains valuable catalyst and is therefore fed to the reaction zone of step (a) of the process.

Preferably, the phase separation of step (b) is caused by cooling the (single-phase) hydroformylation product to a temperature within the range of 0 to 80 °C, more preferably within the range of 15 to 60 °C. However, it is within the reach of those skilled in the art to establish in each case the degree of cooling and the optimal amount of solvent required for phase separation to occur. No specific pressure requirements apply. The experimental results provided hereinafter are also indicative for the amount of solvent preferably to be used.

A further embodiment of the process of the invention relates to phase separation problems. These problems concern the excessively long time required for the layers to separate cleanly and the need for a coolant having a temperature below room temperature in case of phase separation at ambient temperature. The former problem appears due to the formation of a fine emulsion, the latter to the relationship between temperature and the catalyst distribution over both layers. Note that the second problem has already been partly addressed by the process of the invention as the catalyst dissolved in the product layer is recovered by extraction.

The inventors found that use of a centrifuge, a filterbed coalescer or an electrostatic coalescer in step (b) of the process will break the emulsion in a highly expedient manner without loss of valuable material. On the other hand, the use of conventional equipment such as an open settler, a parallel-plate settler and a hydrocyclone have proved to be less successful (for definitions on equipment to break emulsions cf. "Perry's Chemical Engineers' Handbook", 6th ed., pp. 21-64; 21-65 and 21-66).

As in EP-A-0,350,922, it has been found helpful to add liquid saturated hydrocarbons to the crude hydroformylation product before phase separation. Preferably, these hydrocarbons have boiling points equal to or less than the light ends (i.e., paraffin byproducts produced in the process). Indeed, the light ends themselves may be used. Benefits include accelerated phase separation, even at higher temperatures. This allows for use of less cold and hence less expensive coolants. Further benefits include improved extraction (as the catalyst is typically less soluble in saturated hydrocarbons) and washing of the extracted hydroformylation product. Conveniently these hydrocarbons are used in an amount of 10 to 50 percent by weight based on the product stream.

In addition to the main process as described above, it was found beneficial to recycle a portion of the hydroformylation product to the reaction zone, to improve the dissolution of the ethylenically unsaturated compounds in the solvent. This is particularly advantageous during start-up. Conveniently, up to 20 per cent of the hydroformylation product may so be recycled.

The ethylenically unsaturated compounds used as starting material may be compounds having a single double bond. They may bear functional groups attached to their backbone or have non-carbon atoms in their backbone. Preferably, they have from 2 to 30 carbon atoms per molecule. They may also be applied as a mixture of such ethylenically unsaturated compounds. More preferably, the ethylenically unsaturated compound is an olefin having from 4 to 24 carbon atoms per molecule, or it is a mixture of such olefins. It is believed that with olefins having only 2 or 3 carbon atoms per molecule it may be difficult to cause phase separation in step (b) to occur. Most preferred are olefins having from 6 to 18 carbon atoms, or mixtures thereof. Such olefin mixtures are commercially readily available and their products represent valuable detergent and plasticizer intermediates.

Carbon monoxide and hydrogen gas may be supplied in equimolar or non-equimolar ratios, e.g. in a ratio within the range of 5:1 to 1:5. Preferably they are supplied in a ratio within the range of 2:1 to 1:2.5.

The hydroformylation can be suitably carried out at moderate reaction conditions. Hence temperatures in the range of 50 to 200 °C are recommended, preferred temperatures being in the range of 70 to 160 °C.

Reaction pressures in the range of 1 to 300 bar absolute are suitable, but in the range of 5 to 100 bar abs are preferred. Lower or higher pressures may be selected, but are not considered particularly advantageous.

Suitable solvents that are capable of selectively dissolving substantially all of the catalyst system from the hydroformylation product, are characterized by the presence of an aprotic, polar group in the molecule. Solvents containing strong polar groups are in particular preferred if the unsaturated starting material has a relatively low molecular weight, e.g., if ethylenically unsaturated compounds having from 5 to 7 carbon atoms are used. For the hydroformylation of higher molecular weight unsaturated compounds, e.g. olefins having from 12 to 16 carbon atoms the use of less polar solvents will usually be satisfactory.

Solvents comprising or substantially consisting of sulphones are preferred. Solvents that are particularly preferred comprise dialkylsulphones such as dimethyl-sulphone and diethylsulphone; and cyclic sulphones such as sulfolane (tetrahydrothiophene-2,2-dioxide), 2-methylsulfolane and 2-methyl-4-ethyl-sulfolane. A further class of suitable solvents includes the class of compounds having 2 or more cyano groups, such as malononitrile, succinonitrile, adiponitrile, dihydro-muconitrile, pimelonitrile, suberonitrile, 1,6-di-cyanocyclohexane, 1,2,4-tricyanobutane, etc., and mixtures thereof either or not with sulfolane.

Other mixtures of solvents may also be used, for example a mixture of a sulphone and/or a compound having 2 or more cyano groups with a protic solvent, such as an alcohol. In the hydroformylation of olefins, typically an alcohol is selected which is identical or similar to the alcohol obtained in the hydroformylation reaction. Sulfolane is the preferred solvent for the present process.

The extractant is characterized by the presence of an aprotic, polar group in the molecule as well. The compounds mentioned above (as solvent) are therefore suitable extractants. In the present invention, the extractant and solvent are the same.

The amount of solvent to be used in the process of the invention may vary considerably. For instance, the amount of solvent may vary from 3 to 50 percent by volume of the volume of the reaction mixture comprising solvent, ethylenically unsaturated compound(s) and catalyst.

In the present specification the catalyst may be a palladium metal carbonyl and palladium metal carbonyl hydride, modified with one or more noncarbonyl, non-ionically charged phosphorus ligands. Examples of noncarbonyl ligands that already find employ in the manufacture of hydroformylation products include phosphines, phosphine oxides, phosphites and the like. The invention is particularly useful when employing any of the catalysts described in EP-A-0,220,767, US-A-3,527,818, EP-A-0,495,547 and especially when applying the catalysts of WO 95/05354.

A suitable catalyst system, for instance, comprises
(i) a source of palladium metal cations;
(ii) a source of anions, other than halide anions, e.g., derived from acids having a pKa value of less than 3 when measured in an aqueous solution at 18 °C; and
(iii) a source of bidentate ligands of the formula

   R¹R²P-R-PR³R⁴ (I)
wherein R is a bivalent bridging group containing from 1 to 10 atoms in the bridge and R¹, R², R³ and R⁴ independently represent substituted or non-substituted aliphatic groups, or R¹ together with R² and/or R³ together with R⁴ represent a bivalent group with at least 5 ring atoms whereby the two free valencies are linked to the phosphorus atom.

An example of a suitable metal source is palladium(II) acetate.

As anion source, other than halide anions, any compound generating anions may be used. Such compounds may comprise acids or salts thereof; for example, any of the acids mentioned above, which may also participate in the salts of the palladium metal. The anions are preferably derived from strong acids, i.e., acids having a pKa value of less than 3, preferably less than 2 as measured in aqueous solution at 18 °C. The anions derived from these acids are non-coordinating or weakly coordinating with the metals. The stronger the acid, the less the anion coordinates with the metal cation and the higher is the linearity of the hydroformylation product.

Typical examples of suitable anions are anions of phosphoric acid, sulphuric acid, sulfonic acids and halogenated carboxylic acids such as trifluoroacetic acid. Also, complex anions are suitable, such as the anions generated by a combination of a Lewis acid such as BF₃, B(C₆F₅)₃, AlCl₃, SnF₂, Sn(CF₃SO₃)₂, SnCl₂ or GeCl₂, with a protic acid, such as a sulfonic acid, e.g. CF₃SO₃H or CH₃SO₃H or a hydrohalogenic acid such as HF or HCl, or a combination of a Lewis acid with an alcohol. Examples of such complex anions are BF₄⁻, SnCl₃⁻, [SnCl₂.CF₃SO₃]⁻ and PF₆⁻. The preferred anion source is trifluoromethanesulfonic acid.

The bridging group in the diphosphine, represented by R, typically is composed of carbon atoms. Preferably the bridging group contains two or three carbon atoms in the bridge.

In the ligands of formula (I), R¹, R², R³ and R⁴ may independently represent various non-cyclic or cyclic groups, optionally substituted with substituents such as alkoxy groups with 1 to 4 carbon atoms, halogen atoms or (C₁ to C₄ alkyl)amino groups. Examples are alkyl groups such as ethyl, isopropyl, sec-butyl and tert-butyl groups, cycloalkyl groups such as cyclopentyl and cyclohexyl groups, aryl groups such as phenyl and tolyl groups.

However, preferably at least one of R¹ together with R² or R³ together with R⁴ represents a bivalent (substituted) group.

The bivalent (substituted) group preferably contains from 6 to 9 atoms in the ring. More preferably it is a cyclic group containing 8 ring atoms. Substituents, if any, are usually alkyl groups having from 1 to 4 carbon atoms. As a rule, all ring atoms are carbon atoms, but bivalent cyclic groups containing one or two heteroatoms in the ring, such as oxygen or nitrogen atoms are not precluded. Examples of suitable bivalent cyclic groups are 1,4-cyclohexylene, 1,4-cycloheptylene, 1,4-cyclooctylene, 1,5-cyclooctylene, 2-methyl-1,5-cyclooctylene, 2,6-dimethyl-1,4-cyclooctylene and 2,6-dimethyl-1,5-cyclooctylene groups.

Preferred bivalent cyclic groups are selected from 1,4-cyclooctylene, 1,5-cyclooctylene, and methyl (di)substituted derivatives thereof.

Mixtures of ligands comprising different bivalent cyclic groups may be used as well, e.g. mixtures of ligands with 1,4-cyclooctylene and ligands with 1,5-cyclooctylene groups. Preferred bidentate ligands of formula (I) are therefore 1,2-bis(1,4-cyclooctylenephosphino)ethane, 1,2-bis(1,5-cyclooctylenephosphino)ethane and mixtures thereof and 1,3-bis(1,4-cyclooctylenephosphino)propane, 1,3-bis(1,5-cyclooctylenephosphino)propane and mixtures thereof.

For the preparation of the bidentate ligands, reference is made to known techniques, for example the method disclosed in GB-A-1,127,965.

The quantity in which the catalyst system is used, is not critical and may vary within wide limits. Usually amounts in the range of 10⁻⁸ to 10⁻¹, preferably in the range of 10⁻⁷ to 10⁻² mole atom of metal per mole of ethylenically unsaturated compound are used. The amounts of the participants in the catalyst system are conveniently selected such that per mole atom of metal from 0.5 to 10, preferably from 1 to 6 moles of bidentate ligand are used, from 0.5 to 15, preferably from 1 to 8 moles of anion source or a complex anion source.

A preferred feature of the process of the invention consists in the presence of a catalyst promoter, comprising a source of halide anions (Cl, Br or I anions), with the proviso that the molar ratio between halide anions and the metal cations should be at most 5:1. Preferably, the molar ratio between halide anions and metal cations is at most 1:1, for instance from 0.02:1 to 1:1.

As source of halide anions any compound generating halide anions under the reaction conditions may be used.

Recommended are inorganic compounds such as hydrogen halides, e.g. HCl, HBr and HI and metal halides, e.g. NaCl, MgBr₂, ZnCl₂, ZnI₂, KBr, RbCl, CsCl, CsI, MgI₂ and CuCl. Catalyst promoters comprising a source of chloride anions are in particular preferred.

### Figures

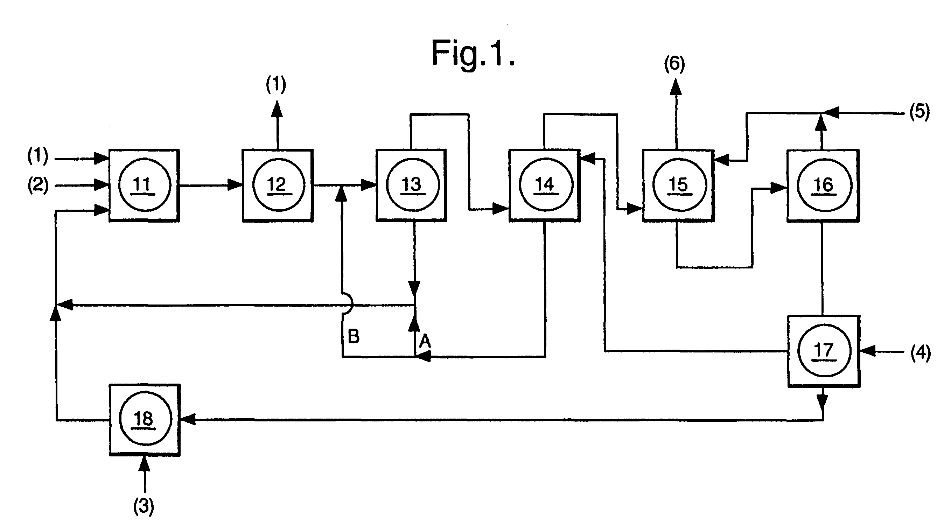

The various embodiments of the process have been shown in the included schematic block diagrams (Figures 1, 2 and 3).

In the Figures, the following legend is used:
- 1: carbon monoxide and hydrogen gas (syngas)
- 2: ethylenically unsaturated compound
- 3: catalyst components
- 4: solvent
- 5: water
- 6: crude alcohol
- 7: light ends
- 8: heavy ends
- 9: finished alcohol
- 10: hydrogen gas
- 11: one or more gas/liquid reactors
- 12: one or more gas/liquid separators
- 13: a centrifuge, coalescer or settler
- 14: solvent extraction column (catalyst removal)
- 15: water extraction column (solvent removal)
- 16: water/solvent distillation column
- 17: intermediate solvent storage
- 18: catalyst preparation
- 19: topping distillation column (light ends)
- 20: tailing distillation column (heavy ends)
- 21: hydrofinishing

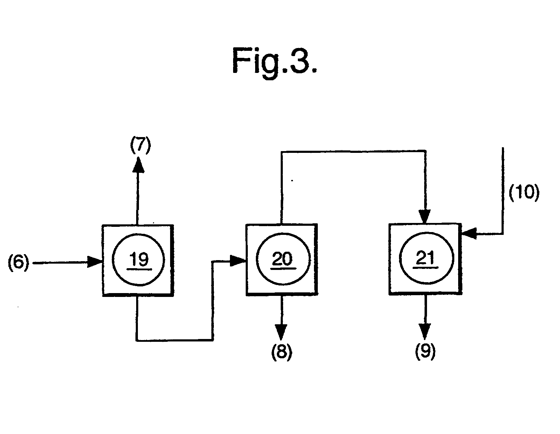

In Figure 1, the ethylenically unsaturated compound (2), carbon monoxide and hydrogen gas (1) are fed into the reactor(s) (11) that contain(s) catalyst prepared in unit (18). The product is passed to one or more gas/liquid separators (12). Thereafter, the product is sent to either of the centrifuge, filterbed coalescer or electrostatic coalescer (13). The solvent layer from (13) is recycled together with the catalyst to the reactor(s) (11). The product layer is passed to the solvent extraction column (14), wherein the remaining catalyst is removed. The loaded solvent from the solvent extraction column (14) is passed through conduits A or B to (11) and/or (13). The product is passed to the water extraction column (15), wherein the solvent is removed. The aqueous layer is passed to distillation column (16), to recover water (5) over the top, which is recycled to (15), and to recover solvent (4) at the bottom of (16), which is recycled to storage (17). The conduits to recycle the water and solvent have inlets for water (5) and solvent (4). The product layer (6) that is recovered from (15) may be passed to any suitable work-up train, for instance, such as disclosed in Figure 3.

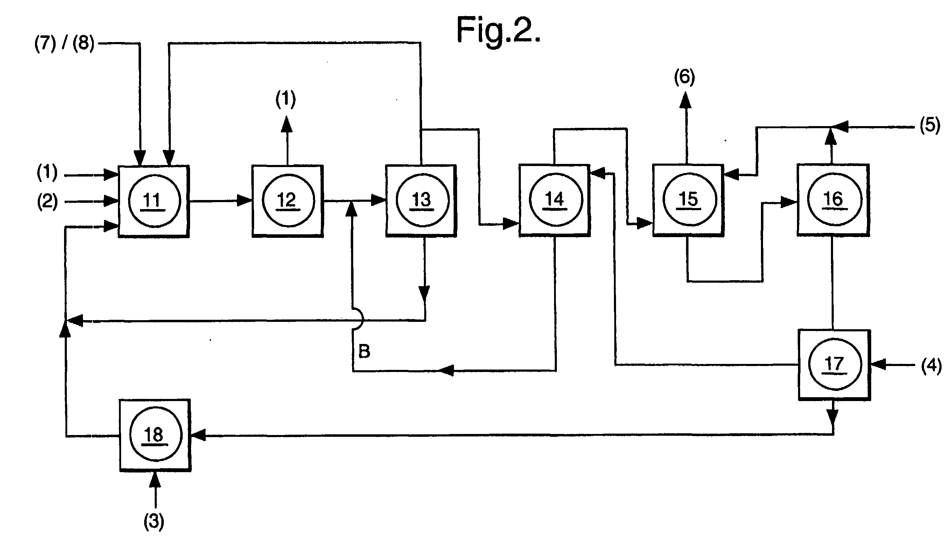

Figure 2 is a representation of a preferred embodiment, wherein the loaded solvent from the solvent extraction column (14) is passed through conduit B to (13). Additionally, this figure depicts the process wherein part of the crude reaction product is recycled to (11).

Finally, Figure 3 shows a suitable work-up train, wherein the product (6) is passed to one or more light end distillation columns (9), wherein additionally the residual water is removed, one or more heavy ends distillation columns (10) and finally a hydrofinishing reactor (11). A saponification unit (not shown) before the distillation columns is optional. Besides, the light ends (7) and/or heavy ends (8), after separation from the water (not shown), may be recycled to reactor (11). This is particularly advantageous when a saturated hydrocarbon is added to facilitate the phase separation step.

The invention will be further illustrated by the following examples. In these examples the following abbreviations are used:
- BCPE =: 1,2-bis(cyclooctylenephosphino)ethane
- TFSA =: trifluoromethanesulphonic acid
- NaCl =: sodium chloride

### Reference example A

Hydroformylation has been carried out in a 300 ml batch autoclave. The autoclave was filled, under argon atmosphere, with 62 ml C₁₁-C₁₂ internal linear olefins, 0.63 ml water and 0.755 g n-tridecane (as GC internal standard). Separately, catalyst was prepared in a 100 ml flask, by adding 0.525 g Pd acetate, 1.01 g BCPE, 0.522 g zinc dichloride and 0.92 g TFSA, under argon atmosphere, to 99.0 g anhydrous sulfolane (40 °C, stirring), to give a Pd concentration of ca. 400 ppmw and molar catalyst ratios (Pd/BCPE/TFSA/chloride) of 1:1.4:2.6:3.2. One sixth of this catalyst inventory (16.96 g) was added to the batch autoclave. Subsequently also 50 ml 2-ethylhexanol was added. The autoclave was closed, residual air was removed using three cycles of vacuum and syngas pressurizing. Stirring rate was 1000 rpm, using a hollow-shaft stirrer. Then the pressure was set at 56 bar syngas (molar ratio H₂/CO of 2.0) and temperature was increased rapidly to 90 °C. At the start of the reaction the temperature rose to 105 °C and the pressure dropped. The reaction was maintained at this temperature and at a pressure of 50 bar (using syngas make-up via a constant pressure valve). After 3 h at 105 °C, the reactor contents were cooled to ambient temperature. GC analysis indicated that olefin conversion after 3 h was >99.9% and selectivities to alcohol, paraffin and heavy ends were 99.0, 0.5 and 0.5%, respectively (no aldehyde observed).

### Reference example B

Part of the product of reference example A was heated to 40 °C (a reasonable model temperature for phase separation in a commercial unit, using tempered cooling water). At this temperature, two liquid phases existed: an upper almost white layer of crude alcohol; and a lower layer of Pd catalyst dissolved in sulfolane (yellow due of presence of Pd catalyst). The layers were separated, sampled, diluted in n-butanol and analyzed by elemental analysis. The data were as follows:

| Layer (g) | Pd (mg/kg) | P (mg/kg) | Cl (mg/kg) | F (mg/kg) |
|---|---|---|---|---|
| alcohol 93.2 | 122 | 128 | 224 | 306 |
| sulfolane 4.0 | 4110 | 3288 | 1950 | 2883 |
| partition coefficients | 34 | 26 | 9 | 9 |

Apparently, the Pd catalyst recovery was far from complete. Although for Pd the partitioning coefficient was highest, mass balance indicated that only 59% of the Pd was recovered via the sulfolane layer.

### Reference example C

The experiment described under A was repeated using a 40:60 w/w C₁₂/C₁₃ detergent alcohol, ("DOBANOL" 23, trade mark, linearity ca. 80%), instead of 2-ethylhexanol. The molar catalyst ratios (Pd/BCPE/TFSA/Cl) were as follows: 1:1.4:2.0:3.2. Olefin conversion after 2 h and 4 h was 94.6 and > 99.9 %, respectively. Selectivities after 4h were 99.5% alcohol, 0.4% paraffin, 0.1% aldehyde (no heavy ends detected). After 4 h reaction time, the product was cooled to 60 °C (still one phase) and split in three nearly equal fractions. These fractions were cooled further to 25, 35 and 45 °C, resulting in phase separation in all three cases. After sufficient equilibration time, the layers were separated and samples were taken from both layers and diluted with methanol and analyzed for Pd. The partitioning coefficients for Pd at 25, 35 and 45 °C were 64, 51 and 35, respectively. These data are consistent with the data of example B. Again, Pd catalyst recovery was far from complete and although the distribution improved with decreasing temperature, even around ambient temperature significant amounts of Pd were lost via the alcohol phase. The mass balance at 25 °C indicated that only 85% of the Pd was recovered via the sulfolane layer.

### Example 1

To demonstrate the advantages of the invention, an experiment was run in a continuously operating mini-pilot-plant consisting of a feed zone, a reactor zone, a first phase separation zone for first catalyst recycling, and an extraction zone for essentially complete recovery and recycling of Pd catalyst and sulfolane.

The feed zone supplied C₁₁-C₁₂ internal linear olefin (purified over alumina to remove peroxides), H₂ (purified over Cu/alumina to remove oxygen), CO (purified over Cu/alumina to remove oxygen and activated carbon to remove carbonyls), BCPE (dissolved in toluene), and water. Before start-up, an inventory of Pd-catalyst (2.7 g Pd, molar ratio Pd/BCPE/TFSA/Cl 1 : 1.1 : 2.0 : 0.43, at a concentration of 3000 ppmw Pd in sulfolane containing 3%w water) was added to the reactor zone. Of the catalyst system only BCPE is made up during the experiment.

The reaction zone consists of two 1.5 litre Inconel-600 Continuous Stirred Tank Reactors (stirrer rate 800 rpm, liquid volume 900 ml each). Reaction conditions were typically 105 °C, 70 bar syngas (H₂/CO molar ratio 2.0), 150-300 g/h olefin feed, 15%w sulfolane, 1.6 %w water, 300-400 ppmw Pd in reactors, olefin conversion in first and second reactor ca. 86-93% and 97.5-99.5%, respectively. After the second reactor, the reactor product was cooled to 35-40 °C and depressurized to 1.2 bar.

Phase separation started directly but was not complete. The two-phase mixture was subsequently sent down-flow through a small filterbed-coalescer (35-40 °C), consisting of flat stainless-steel filter elements containing fibres increasing gradually in size from 2 mm to 22 mm going from inlet to outlet. The product was fully phase-separated and introduced into a settler, from where the sulfolane phase (containing concentrated Pd catalyst) was recycled back to the reactors and the alcohol phase (still containing significant amounts of diluted Pd catalyst and sulfolane solvent) was transported to a separate alcohol buffer vessel.

In the extraction zone, the alcohol phase was purified in two counter-current liquid/liquid extraction columns (glass, 3 cm diameter, 3.4 m height, stainless steel internals of size 4x4 mm). In the sulfolane extraction column, clean recycle sulfolane was used to remove essentially all Pd residues from the alcohol (phase ratio alcohol/sulfolane ca. 9:1 w/w, temperature 40 °C, sulfolane as continuous phase). In the water extraction column, clean recycle water was used to remove essentially all sulfolane from the alcohol (phase ratio alcohol/water ca. 5:2 w/w, temperature 70 °C, water as continuous phase). The catalyst-loaded sulfolane from the sulfolane extraction column was recycled back to the filterbed coalescer. The sulfolane-loaded water was fed to a distillation column producing a clean water top product (recycled to water extraction) and a sulfolane bottom product (containing some residual water, ca. 3 %w), which was recycled back via a buffer vessel to the sulfolane extraction column. The crude alcohol coming from the mini-pilot-plant was virtually Pd-catalyst- and sulfolane-free.

Typical Pd concentrations during steady-state were: 400 ppmw in reactors, 2600 ppmw in sulfolane recycle, 90 ppmw in crude alcohol product from filterbed coalescer, 900 ppmw in sulfolane bottoms product of sulfolane extraction, 0.2 ppmw in alcohol top product of sulfolane extraction. The other catalyst components roughly behave as the Pd: typical residual levels of phosphorous, TFSA and chloride in the alcohol top product of sulfolane extraction are: 10 ppmw, < 5 ppmw and < 10 ppmw, respectively. Typical sulfolane concentrations during steady-state were: 15%w in reactors, 8-10%w sulfolane (saturation) in alcohol phases up front of water extraction column, 40 ppmw in alcohol top product of water extraction column.

The run has continued for 732 h and has proven, besides good catalyst retention, also excellent chemical catalyst stability.

### Example 2

Model experiments were conducted to determine the efficiency of water extraction to recover sulfolane from representative product alcohols. The starting mixture was composed of "DOBANOL" 23 (trade mark) and sulfolane in ratio 93/7 w/w. To this mixture, 10 or 30%w water was added at four different temperatures (35, 70, 80, 90 °C). The mixtures were stirred for equilibration and sufficient time was taking for subsequent settling. The resulting two layers (alcohol tops, water bottoms) were separated and analyzed for sulfolane in both layers by GC. The following distribution coefficients were obtained.

| Temperature (°C) | water fraction added (%w on alcohol/sulfolane) | distribution coefficient of sulfolane (bottoms/top w/w) |
|---|---|---|
| 35 | 10 | 8.3 |
| 35 | 30 | 8.2 |
| 70 | 10 | 7.5 |
| 70 | 30 | 5.9 |
| 80 | 10 | 5.5 |
| 80 | 30 | 5.4 |
| 90 | 10 | 3.7 |
| 90 | 30 | 4.8 |

It is clear that sulfolane preferably goes to the water layer, but that many stages are required to reduce the sulfolane concentration to the desired low levels. Temperature has a negative influence on separation efficiency.

### Example 3

Experiments were carried out to check the effect of additives to improve the Pd distribution coefficient over sulfolane and alcohol phases. For this purpose, batch autoclave experiments were carried out in a 300 ml batch autoclave, as described above under Examples A and C. The autoclave was filled with C₆-C₈ or C₁₁-C₁₂ internal linear olefins(I or II respectively), water and n-tridecane (as GC internal standard). Catalyst was prepared using sodium iodide in case of C₆-C₈ olefins and sodium chloride in case of C₁₁-C₁₂ olefins (giving a Pd concentration of ca. 400 ppmw and molar catalyst ratio, Pd/BCPE/TFSA/halide, of 1:1.4:2.0:0.4 in anhydrous sulfolane). Subsequently also product alcohol (ca. 40%w) was added. The reaction was carried out at 105 °C and 50 bar. After 2 h at 105 °C and full conversion, the reactor contents were cooled to ambient temperature. Next, the product was mixed with varying amounts of n-heptane (0-50%w) and equilibrated at the selected temperature. Subsequently, both layers were samples and analyzed for Pd concentration using AAS, as follows:

| alcohol derived from (olefin): | I | I | I | II | II |
|---|---|---|---|---|---|
| n-heptane conc. (%w) | 0 | 33 | 50 | 0 | 50 |
| temperature (°C) | 20 | 20 | 20 | 35 | 35 |
| Pd conc. in top layer (ppmw) | 113 | 23 | 11 | 40 | 12 |
| Pd conc. in bottoms layer (ppmw) | 3320 | 2790 | 2710 | 3320 | 3050 |
| Pd distribution coefficient (bottoms/top w/w) | 29 | 123 | 255 | 80 | 263 |

It is clear that addition of n-heptane has a beneficial effect on Pd recovery.

### Example 4

In order to obtain efficient and fast phase separation, initially a batch centrifuge was used, Subsequently, trials were carried out with a continuous Alfa-Laval LAB 102B-05 centrifuge. This centrifuge was equipped with a so-called purifier-bowl, the type of bowl needed for a liquid-liquid separation, with two liquid outlets. Experiments were carried out with well-mixed feeds of "DOBANOL" 23 (trademark) and Pd-catalyst-containing sulfolane (13.3 %w sulfolane, balance being the alcohol) at a speed of 1500 rpm, 35-50 °C, using flows of 125-850 ml/min. In all cases, separation was rapid and complete as could be seen from the absence of any haze (residual sulfolane droplets) as well as the absence of any (Pd-catalyst-related) yellow colour in the alcohol phase.

## Claims

1. A hydroformylation process including the steps of:
(a) contacting one or more ethylenically unsaturated compound(s) in a reaction zone with carbon monoxide and hydrogen gas in the presence of a polar aprotic solvent and a homogeneous catalyst based on palladium, and a non-ionically charged phosphorus ligand to form a crude hydroformylation product;
(b) allowing a major portion of the solvent, wherein a major portion of the catalyst is dissolved, to separate and be withdrawn from the crude hydroformylation product in a phase separation zone;
(c) removing from the separated hydroformylation product emerging from step (b) substantially all remaining catalyst dissolved therein with a non-aqueous extractant in an extraction zone;
wherein the separated solvent containing catalyst emerging from step (b) is fed to the reaction zone of step (a) and
wherein the non-aqueous extractant used in step (c) is the same substance as the solvent used in step (a).

2. A hydroformylation process according to claim 1 wherein the catalyst-loaded extractant from step (c) is led to the reaction zone of step (a) and/or to the separation zone of step (b).

3. A hydroformylation process according to claim 1 or 2, wherein the amount of extractant used in step (c) is between 0.9 and 1.1 times the amount of solvent that is dissolved in the hydroformylation product.

4. A hydroformylation process according to any of claims 1 to 3, wherein the following step is added:
(d) removing from the extracted hydroformylation product emerging from step (c) substantially all remaining solvent and/or extractant dissolved therein with a washing medium, that is preferably water, in a washing zone, separating the solvent and/or extractant from the washing medium emerging from the washing zone and optionally reusing each.

5. A hydroformylation process according to any of claims 1 to 4, wherein the extraction of step (c) is conducted in a column equipped with a rotating disk contactor or a packed bed column.

6. A hydroformylation process according to any of claims 1 to 5 wherein in step (b) the crude hydroformylation product is cooled to a temperature within the range of 0 to 80 °C.

7. A hydroformylation process according to any of claims 1 to 6, wherein the phase separation of step (b) is assisted by the use of a centrifuge, a filterbed coalescer or an electrostatic coalescer.

8. A hydroformylation process as claimed in any of claims 1 to 7, wherein liquid saturated hydrocarbons are fed to the phase separation zone of step (b).

9. A hydroformylation process according to any of claims 1 to 8, wherein a portion of the hydroformylation product emerging from step (b) is recycled to the reaction zone.

10. A hydroformylation process according to any of claims 1 to 9, wherein the solvent is a sulphone, a compound having 2 or more cyano groups, or a mixture comprising the sulphone and/or the compound having 2 or more cyano groups together with a protic solvent, the ethylenically unsaturated compound is an olefin or a mixture of olefins having from 4 to 24 carbon atoms per molecule, the carbon monoxide and hydrogen gas are supplied in a ratio within 5:1 to 1:5, and wherein the hydroformylation process is carried out at a temperature in the range of 50 to 200 °C and at a pressure in the range of 1 to 300 bar absolute.

## Patentansprüche

1. Hydroformylierungsverfahren, das die folgenden Schritte umfaßt:
(a) Inkontaktbringen einer oder mehrerer ethylenisch ungesättigter Verbindung(en) mit Kohlenmonoxid und Wasserstoffgas in einer Reaktionszone in Gegenwart eines polaren aprotischen Lösungsmittels und eines homogenen Katalysators auf Palladiumbasis und eines nichtionisch geladenen Phosphorliganden zur Ausbildung eines Hydroformylierungsrohproduktes;
(b) Abtrennenlassen eines Hauptteiles des Lösungsmittels, worin ein Hauptanteil.des Katalysators gelöst ist, von dem Hydroformylierungsrohprodukt in einer Phasentrennzone und Abziehen dieser Lösungsmittelhauptmenge;
(c) Entfernen von im wesentlichen dem gesamten, im aus Stufe (b) kommenden, abgetrennten Hydroformylierungsprodukt verbliebenen, darin gelösten Katalysator mit einem nicht-wäßrigen Extraktionsmittel in einer Extraktionszone;
worin das aus Stufe (b) kommende abgetrennte, katalysatorhältige Lösungsmittel der Reaktionszone von Stufe (a) zugespeist wird und worin das in Stufe (c) eingesetzte nicht-wäßrige Extraktionsmittel die gleiche Substanz wie das in Stufe (a) eingesetzte Lösungsmittel ist.

2. Hydroformylierungsverfahren nach Anspruch 1, worin das mit Katalysator beladene Extraktionsmittel aus Stufe (c) zur Reaktionszone von Stufe (a) und/oder zur Trennzone von Stufe (b) geführt wird.

3. Hydroformylierungsverfahren nach Anspruch 1 oder 2, worin die in Stufe (c) eingesetzte Menge an Extraktionsmittel zwischen dem 0,9- und 1,1-fachen der Lösungsmittelmenge liegt, die in dem Hydroformylierungsprodukt gelöst ist.

4. Hydroformylierungsverfahren nach einem der Ansprüche 1 bis 3, worin die folgende Stufe angeschlossen wird:
(d) Abtrennen aus dem aus Stufe (c) kommenden extrahierten Hydroformylierungsprodukt von im wesentlichen dem gesamten darin gelösten verbliebenen Lösungsmittel und/oder Extraktionsmittel mit einem Waschmedium, das vorzugsweise Wasser ist, in einer Waschzone, Abtrennen des Lösungsmittels und/oder Extraktionsmittels von dem aus der Waschzone kommenden Waschmedium und gegebenenfalls jeweiliges Wiederverwenden.

5. Hydroformylierungsverfahren nach einem der Ansprüche 1 bis 4, worin die Extraktion von Stufe (c) in einer mit einem rotierenden Scheibenkontaktor ausgestatteten Kolonne oder in einer gepackten Kolonne ausgeführt wird.

6. Hydroformylierungsverfahren nach einem der Ansprüche 1 bis 5, wobei in Stufe (b) das Hdroformylierungsrohprodukt auf eine Temperatur im Bereich von 0 bis 80°C gekühlt wird.

7. Hydroformylierungsverfahren nach einem der Ansprüche 1 bis 6, worin die Phasentrennung von Stufe (b) durch die Anwendung einer Zentrifuge, eines Filterbettkoaleszierers oder eines elektrostatischen Koaleszierers unterstützt wird.

8. Hydroformylierungsverfahren nach einem der Ansprüche 1 bis 7, worin zu der Phasentrennzone von Stufe (b) flüssige, gesättigte Kohlenwasserstoffe zugespeist werden.

9. Hydroformylierungsverfahren nach einem der Ansprüche 1 bis 8, worin ein Teil des aus Stufe (b) kommenden Hydroformylierungsproduktes zu der Reaktionszone rezykliert wird.

10. Hydroformylierungsverfahren nach einem der Ansprüche 1 bis 9, worin das Lösungsmittel ein Sulfon, eine zwei oder mehr Cyanogruppen aufweisende Verbindung oder ein Gemisch ist, das das Sulfon und/oder die zwei oder mehr Cyanogruppen aufweisende Verbindung zusammen mit einem protischen Lösungsmittel umfaßt, die ethylenisch ungesättigte Verbindung ein Olefin oder ein Gemisch von Olefinen mit 4 bis 24 Kohlenstoffatomen pro Molekül ist, das Kohlenmonoxid und Wasserstoffgas in einem Verhältnis innerhalb eines Bereiches von 5:1 bis 1:5 zugeführt werden und worin das Hydroformylierungsverfahren bei einer Temperatur im Bereich von 50 bis 200°C und bei einem Druck im Bereich von 1 bis 300 bar absolut ausgeführt wird.

## Revendications

1. Procédé d'hydroformylation qui comprend les étapes qui consistent à:
(a) dans une zone de réaction, mettre en contact un ou plusieurs composés éthyléniquement insaturés avec un gaz de monoxyde de carbone et d'hydrogène en présence d'un solvant polaire non protoné et d'un catalyseur homogène à base de palladium et d'un ligand phosphore sans charge ionique pour former un produit brut d'hydroformylation;
(b) dans une zone de séparation de phases, amener une majeure partie du solvant dans laquelle une majeure partie du catalyseur est dissoute à se séparer et à être extraite du produit brut d'hydroformylation et
(c) du produit d'hydroformylation séparé qui provient de l'étape (b), extraire essentiellement tout le reste du catalyseur qui y est dissous à l'aide d'un extradant non aqueux dans une zone d'extraction,
le solvant séparé qui contient le catalyseur et qui provient de l'étape (b) étant injecté dans la zone de réaction de l'étape (a) et l'extractant non aqueux utilisé dans l'étape (c) étant la même substance que le solvant utilisé dans l'étape (a).

2. Procédé d'hydroformylation selon la revendication 1, dans lequel l'extractant chargé en catalyseur qui provient de l'étape (c) est envoyé dans la zone de réactions de l'étape (a) et/ou dans la zone de séparation de l'étape (b).

3. Procédé d'hydroformylation selon les revendications 1 ou 2, dans lequel la quantité d'extractant utilisé dans l'étape (c) est comprise 0,9 et 1,1 fois la quantité de solvant qui est dissoute dans le produit d'hydroformylation.

4. Procédé d'hydroformylation selon l'une quelconque des revendications 1 à 3, dans lequel on ajoute l'étape suivante :
(d) à l'aide d'un fluide de lavage qui est de préférence l'eau et dans une zone de lavage, enlèvement d'essentiellement tous les restes de solvant et/ou d'extradant qui sont dissous dans le produit d'hydroformylation extrait qui provient de l'étape (c), pour séparer le solvant et/ou l'extractant du milieu de lavage qui provient de la zone de lavage et facultativement réutiliser le solvant et/ou l'extractant.

5. Procédé d'hydroformylation selon l'une quelconque des revendications 1 à 4, dans lequel l'extraction de l'étape (c) est conduite dans une colonne équipée d'un contacteur à disque rotatif ou dans une colonne à lits empilés.

6. Procédé d'hydroformylation selon l'une quelconque des revendications 1 à 5, dans lequel, dans l'étape (b), le produit d'hydroformylation est refroidi à une température comprise dans la plage de 0 à 80° C.

7. Procédé d'hydroformylation selon l'une quelconque des revendications 1 à 6, dans lequel la séparation des phases de l'étape (b) est assistée par recours à une centrifugeuse, à un coalesceur à lit de filtration ou à un coalesceur hydrostatique.

8. Procédé d'hydroformylation selon l'une quelconque des revendications 1 à 7, dans lequel des hydrocarbures saturés liquides sont introduits dans la zone de séparation des phases de l'étape (b).

9. Procédé d'hydroformylation selon l'une quelconque des revendications de 1 à 8, dans lequel une partie du produit d'hydroformylation qui provient de l'étape (b) est renvoyée dans la zone de réaction.

10. Procédé d'hydroformylation selon l'une quelconque des revendications 1 à 9, dans lequel le solvant est un(e?) sulfone, un composé qui contient 2 ou plusieurs groupes cyano ou un mélange qui comprend le sulfone et/ou le composé qui contient 2 ou plusieurs groupes cyano en même temps qu'un solvant protoné, le composé éthyléniquement insaturé étant une oléfine ou un mélange d'oléfines qui compte de 4 à 24 atomes de carbone par molécule, les gaz de monoxyde de carbone et hydrogène étant délivrés dans un rapport compris entre 5:1 à 1:5, le procédé d'hydroformylation étant conduit à une température comprise dans la plage de 50 à 200° C et à une pression de comprise dans la plage de 1 à 300 bars absolus.
